# FASCICULE DE BREVET EUROPEEN

(11) **EP 0 722 952 B1**
(45) Date de publication et mention de la délivrance du brevet: **02.01.2004**
(21) Numéro de dépôt: 96490005.4
(22) Date de dépôt: 19.01.1996
(51) Int. Cl.: C07K 16/04, A23C 9/20, A61K 35/20, A61K 39/395

(54) **Fraction sérocolostrale hypoallergénique à forte activité anticorps, utilisation et procédé de fabrication d'une telle fraction**
Hypoallergenische Serokolostrahlfraktion mit hoher Antikörperaktivität, Verwendung und Verfahren zur Herstellung solcher Fraktion
Hypoallergenic serocolostral fraction having high antibody activity, use and process for preparing such a fraction

(30) Priorité: 20.01.1995 FR 9500854
(43) Date de publication de la demande: 24.07.1996
(73) Titulaire: INGREDIA, 62033 Arras Cedex (FR)
(72) Inventeur: Lefranc-Millot, Catherine, F-59650 Villeneuve D'Ascq (FR); Canivez, Olivier, F-59000 Lille (FR); Quinque, Bernard, F-62140 Marconnelle (FR)
(74) Mandataire: Hennion, Jean-Claude

(56) Documents cités:
- EP-A- 0 320 152
- EP-A- 0 338 229
- EP-A- 0 398 802
- PATENT ABSTRACTS OF JAPAN vol. 009, no. 211 (C-300), 29 Août 1985 & JP-A-60 075433 (ZENKOKU NOUGIYOU KIYOUDOUKUMIAI RENGOUKAI), 27 Avril 1985,
- CHEMICAL ABSTRACTS, vol. 86, no. 1, 3 Janvier 1977 Columbus, Ohio, US; abstract no. 3382n, Y. KUMANO ET AL.: "DEGRADATION OF BOVINE IgG1-IMMUNOGLOBULIN BY RENNIN AND PEPSIN." page 304; XP002001402 & NIPPON CHIKUSAN GAKKAI-HO, vol. 47, no. 9, 1976, pages 543-550,
- LEFRANC-MILLOT ET AL: 'Comparison of the IgE titers to bovine colostral immunoglobulins and their F(ab')2 fragments in sera of patients allergic to milk' ALLERGY AND IMMUNOLOGY vol. 110, 1996, pages 156 - 162

## Description

La présente invention concerne une fraction du colostrum qui est hypoallergénique et qui présente une activité anticorps importante. Elle concerne plus particulièrement une fraction sérocolostrale c'est-à-dire une fraction du colostrum dont a extrait les caséines et les matières grasses. La présente invention concerne également les utilisations d'une telle fraction ainsi que les procédés de fabrication spécialement conçus pour l'obtenir.

Le colostrum désigne de manière générale le mélange de sécrétions lactées et de constituants du sérum sanguin, obtenus dans les sept jours suivant la mise bas (post-partum). Il provient de la vache ou d'autres mammifères.

S'agissant du colostrum bovin, celui-ci est comparativement au lait, beaucoup plus riche en protéines , notamment caséines immunoglobulines, β-lactoglobuline, α-lactalbumine, sérumalbumines.

Depuis longtemps on a pensé utiliser les immunoglobulines présentes dans le colostrum comme anticorps contre un large spectre d'antigènes bactériens, viraux ou parasitaires. Cette activité anticorps est d'ailleurs accrue lorsque le colostrum provient de vaches hyper immunisées, par exemple contre E.coli.

Notamment le document WO 92/O2538 décrit la préparation , par ultra-filtration d'un sérocolostrum hyperimmum contre E.coli , d'un produit contenant des molécules de masse moléculaire supérieure ou égale à 20.000 daltons. Cette préparation exerce un effet préventif sur l'affection à E.coli en réduisant le délai d'excrétion des bactéries par les animaux et en diminuant la mortalité dans le groupe traité.

Le document EP-A-0.338.229 décrit une préparation immunoglobulinique de colostrum bovin pasteurisé, écrémé et décaséiné, obtenue par séchage par pulvérisation du petit lait colostral, destinée au traitement de maladies d'origine bactérienne ou toxinique.

Le document Patent Abstracts of Japan, vol 009, n° 211 (C-300), 29 août 1985 & JP-A-60075433 (Zenkoku Nougiyou Kiyoudoukumiai Rengoudai) 27 avril 1985 décrit un procédé de concentration en immunoglobulines d'un colostrum bovin dégraissé, par addition de pepsine ou de rénine , suivie d'une étape d'ultrafiltration. La fraction ainsi obtenue est séchée par congélation ou pulvérisation pour donner une matière première utilisée dans la préparation d'aliments pour jeunes animaux.

Cependant selon le demandeur les préparations citées dans les documents ci-dessus présentent l'inconvénient majeur d'être allergéniques.

Le but que s'est fixé le demandeur est de proposer une fraction séro-colostrale qui soit hypoallergénique et qui ait une forte activité anticorps.

Ce but est parfaitement atteint par la fraction sérocolostrale de l'invention qui est exempte de β-lactoglobuline , de α-lactalbumine, de sérum-albumine et de fragments Fc des immunoglobulines G (IgG) et dont le nombre de moles d'éléments à activité anticorps pour 100 g de protéines totales est d'au moins 2,5.10⁻⁴.

En effet la β-lactoglobuline est la protéine allergénique majeure puisqu'elle déclenche une réaction de sensibilité chez 82% des patients allergiques au lait, testés avec cette protéine purifiée. Elle est particulièrement dangereuse pour les enfants génétiquement prédisposés.

Bien que leur caractère d'allergénicité soit nettement inférieur à celui de la β-lactoglobuline, il est maintenant admis que l'α-lactalbumine, la sérumalbumine et également les fragments Fc des IgG présentent aussi un certain caractère d'allergénicité. L'élimination des fragments Fc des IgG n'est cependant pas rédhibitoire au regard de l'activité anticorps des IgG, cette activité étant portée principalement par les fragments F(ab')2.

Les éléments à activité anticorps étant les fragments F(ab')2 des (IgG), le nombre de mole de F(ab')2 par 100 g de protéines totales est d'au moins 2,5 10⁻⁴.

La fraction sérocolostrale de l'invention peut se présenter sous forme d'une préparation liquide, éventuellement congelée. Elle peut, préférentiellement , se présenter sous forme de poudre, obtenue notamment à partir de la préparation liquide par atomisation ou éventuellement lyophilisation.

De manière générale la fraction sérocolostrale de l'invention est utilisable comme complément nutritionnel , notamment dans le domaine de la diététique.

Plus spécifiquement la fraction sérocolostrale particulièrement hypoallergénique dont les éléments à activité anticorps sont des fragments F(ab')2 d'IgG est utilisable pour l 'obtention d'un médicament destiné à la prophylaxie ou au traitement, par voie orale, des diarrhées animales ou humaines, que ces diarrhées soient d'origine bactérienne, virale ou parasitaire.

C'est un autre objet de l'invention que de proposer un procédé spécialement conçu pour la préparation de la fraction sérocolostrale de l'invention.

Le procédé consiste à extraire les caséines d'un colostrum bovin écrémé , à faire une hydrolyse contrôlée du sérocolostrum ainsi obtenu par la pepsine , et à adsorber la β-lactoglobuline contenue dans l' hydrolysat par mise en contact de celui-ci sous agitation pendant une durée prédéterminée , avec une résine échangeuse d'ions , de type anionique.

Certes par le document EP-A-320 152 on a déjà proposé de mettre un concentré protéique en contact avec une résine échangeuse d'anions ; mais dans ce cas il s'agissait d'un concentré protéique liquide de lactosérum ou une poudre reconstituée de concentré protéique de lactosérum ; de plus les conditions opératoires et la résine étaient déterminées en sorte d'obtenir avant tout une concentration plus élevée en immunoglobuline. Les figures 4 et 5 de ce document EP-A-320 152 illustrent les profils chromatographiques réalisés à partir du lactosérum de départ (figure 4) et du produit final (figure 5) après dégraissage, traitement par la résine échangeuses d'anions , concentration et séchage. Il ressort de l'examen de la figure 5 que le produit final contient encore de la β-lactoglobuline. Au contraire , dans le procédé de la présente invention, la résine échangeuse d'anions et les conditions opératoires sont déterminées pour obtenir une adsorption totale de la β-lactoglobuline dans le sérocolostrum obtenu après extraction des caséines du colostrum bovin écrémé, éventuellement dilué. Par ailleurs , il est à souligner que dans ce document le nombre de moles d'IgG pour 100 g de protéines totales est de 1,13.10⁻⁴ (tableau 1 de l'exemple 2) , comparativement au 4,5.10⁻⁴ qui est le seuil minimal pour la fraction sérocolostrale de l'invention , lorsqu'il s'agit d'IgG.

L'opération d'adsorption de la β-lactoglobuline par mise en contact avec la résine anionique est réalisée en maintenant le pH à une valeur de l'ordre de 6 , notamment par addition d'acide chlorhydrique.

La quantité de résine anionique qui est utilisée est préférentiellement de l'ordre de 1 Kg de résine humide pour 2,5 litres de sérocolostrum ou pour 2 litres d'hydrolysat de sérocolostrum à environ 50 g par litre de matière sèche.

Préférentiellement l'étape d'hydrolyse ménagée du sérocolostrum se déroule dans les conditions opératoires suivantes :
- pH régulé entre 3,5 et 4,5
- température comprise entre 30 et 40°C
- durée comprise entre 3 et 8 heures
- quantité de pepsine , mesurée en unité SI, par milligramme de protéine, comprise entre 1033 et 3400 (entre 31 et 102 unités Anson).

Ces conditions opératoires spécifiques ont été retenues par le demandeur comme permettant d'obtenir un rendement d'hydrolyse proche de 100% c'est-à-dire une hydrolyse totale des immunoglobulines G , sans présenter de dégradation des fragments F(ab')2.

La sélection des conditions opératoires précises est fonction du choix initial d'un des paramètres.

Dans un exemple préféré de réalisation, les conditions opératoires retenues étaient les suivantes : pH 3,8 ; 37°C pendant 4 heures avec une quantité de pepsine correspondant à 1700 unités SI (51 unités Anson) par milligramme de protéines.

La présente invention sera mieux comprise à la lecture de la description qui va être faite d'exemples de réalisation de fraction sérocolostrale hypoallergénique et à forte activité anticorps.

Partant d'un colostrum bovin écrémé, on obtient le sérocolostrum soit par précipitation acide des caséines soit par action de la présure. Dans un premier exemple de réalisation, partant de 39,6 kg de colostrum bovin écrémé, la précipitation acide des caséines a été menée par dilution au 1/7^{ème} du colostrum et abaissement du pH à 3,8 pendant 10 mn, ce qui permet d'obtenir une décantation rapide et un caillé compact. Cette précipitation a été suivie d'une remontée du pH jusqu'à pH 4,6 de manière à respecter l'intégrité immunologique des immunoglobulines , et suivie d'une incubation pendant 2 heures à 37°C et enfin d'une filtration avec un sac à caillé ayant des mailles de 40 µm.

On a ainsi obtenu 249,9 Kg de sérocolostrum qui ont été concentrés par ultrafiltration tangentielle en mettant en oeuvre des membranes organiques dont le seuil de coupure était de 5 000 daltons jusqu'à atteindre un volume de 59,9 litres.

Selon un second mode de réalisation, on a ajouté à 78,17 litres de colostrum bovin écrémé de la présure à une concentration de 1% en poids , en présence de gluconate de calcium à 6% (P/V) à raison de 5% (V/V) en volume de solution de gluconate par rapport au volume de colostrum. Ce mélange a été suivi d'une incubation pendant 16 heures à 37°C, puis filtration du surnageant avec un sac à caillé permettant d'obtenir 65,5 Kg de sérocolostrum.

Comme cela apparaîtra dans la suite de la présente description, le procédé d'extraction des caséines par la présure donne un meilleur rendement de restitution des immunoglobulines. Dans les deux exemples précités , ce rendement était respectivement de 65% pour la précipitation acide et de 96% pour l'action de la présure. Quant à l'élimination proprement dite des caséines , son efficacité était comparable dans les deux cas.

Le sérocolostrum qui est obtenu soit par précipitation des caséines soit par action de la présure est ensuite traité de manière à obtenir la fraction sérocolostrale de l'invention. Celle-ci se caractérise par une hypoallergénicité certaine et par une forte activité anticorps.

Pour obtenir la fraction sérocolostrale de l'invention à partir du sérocolostrum obtenu conformément au premier et au second exemple précité, le demandeur a mis au point un procédé adapté d'hydrolyse ménagée des immunoglobulines permettant d'obtenir un rendement d'hydrolyse proche de 100%, c'est-à-d'ire une hydrolyse totale des IgG, sans dégradation des fragments F(ab'2. Ce procédé met en oeuvre la pepsine à raison d'une quantité variant entre 1033 et 3400 unités SI (entre 31 et 102 unités Anson) de pepsine par milligramme de protéines , un pH compris entre 3,5 et 4,5 une température variant de 30 à 40°C pendant une durée de 3 à 8 heures.

Certes l'utilisation de la pepsine pour la dégradation de l'immunoglobuline IgG₁ bovin était déjà connue par le document CHEMICAL ABSTRACTS vol. 86 no. 1 , 3 janvier 1977, Colombus, Ohio, US, abstract no. 3382n, mais il s'agissait alors d'hydrolyse de molécules préalablement purifiées. Dans le cas présent le sérocolostrum de départ comprend encore toutes les autres protéines du colostrum à l'exception des caséines. C'est cette particularité du composant de départ ainsi que la recherche d'un rendement optimum d'hydrolyse qui soit de plus sans dégradation des fragments F(ab')2 qui a conduit à la sélection des conditions opératoires précisées ci-dessus.

Dans un exemple précis de réalisation, l'hydrolyse a été menée sur le sérocolostrum dans un réacteur de 100 litres à pH 3,8 ajusté par HC1, et à 37°C pendant une durée de 4 heures , avec une quantité de pepsine correspondant à 1700 unités SI (51 unités Anson) par milligramme de protéines. Le contrôle du pH est réalisé à l'aide d'un pH-stat, avec HC1 1N et l'hydrolyse est arrêtée par remontée du pH final à 8.

Quelque soit le sérocolostrum de départ , obtenu par précipitation acide au par action de la présure du colostrum bovin écrémé , le rendement d'obtention des fragments F(ab')2 est très élevé, compris entre 85 et 100 %. Ce haut rendement est confirmé par électrophorèse en gel de polyacrylamide dans des conditions dénaturantes en présence de dodécyl sulfate de sodium (SDS).

On fait subir au sérocolostrum ainsi hydrolysé, c'est-à-dire à l'hydrolysat un traitement d'adsorption de la β-lactoglobuline par mise en contact avec une résine échangeuse d'ions du type anionique.

Cette adsorption a été réalisée en mettant en contact , sous agitation , dans une cuve le sérocolostrum avec une quantité déterminée de résine échangeuse d'ions de type anionique. Contrairement au procédé décrit dans le document EP-0.398.802 qui concerne l'élimination de lactoglobuline à partir de matières premières contenant des protéines de lactosérum à l'aide d'une résine anionique forte, il s'agit d'une résine anionique faible, du type amine tertiaire , connue sous l'appellation Duolite A 568. Cette mise en contact sous agitation a eu lieu pendant 4 heures en maintenant le pH à une valeur de 6 par addition d'HC1 1N. La quantité de résine était de l'ordre de 1 Kg de résine humide pour 2 litres d'hydrolysat à environ 50 g de protéines par litre. De plus, dans le cas où l'hydrolysat provient du sérocolostrum obtenu par action de la présure, le traitement d'adsorption est réalisé après dilution au demi de l'hydrolysat.

Il est à noter que la fraction sérocolostrale qui est ainsi obtenue est particulièrement hypoallergénique puisqu'elle est totalement débarrassée de la β-lactoglobuline et que de plus elle est exempte des fragments Fc des IgG ainsi que de l'α-lactalbumine et de la sérumalbumine , qui présentent également un certain caractère d'allergénicité.

De plus même si une fraction importante des fragments F(ab')2 est adsorbée par la résine anionique, il n'en reste pas moins vrai que l'activité anticorps de cette fraction sérocolostrale , mesurée en nombre de moles de fragments F(ab')2 pour 100 g de protéines totales a été sensiblement augmentée par rapport à la valeur de cette activité dans le colostrum lui-même.

Cette fraction sérocolostrale, à l'état liquide, peut être présentée par exemple sous forme de poudre, en mettant en oeuvre les techniques classiques d'atomisation ou de lyophilisation.

Les tableaux ci-après illustrent les résultats obtenus à chaque étape du procédé de fabrication, selon les deux voies envisagées ci-dessus, à savoir :
Tableau 1: dilution et précipitation acide des caséines. Concentration par ultrafiltration. Hydrolyse ménagée par la pepsine. Adsorption de la β-lactoglobuline par résine anionique.
Tableau 2: extraction des caséines par la présure. Hydrolyse ménagée par la pepsine. Dilution puis adsorption de la β-lactoglobuline par résine anionique.

Dans tous les cas les tableaux ont été complétés par une étape ultérieure de séchage par atomisation, transformant la fraction sérocolostrale liquide en poudre.

Chaque tableau donne en regard du produit de départ ou intermédiaire ou final les valeurs chiffrées relatives à la quantité, aux matières sèches , aux éléments à activité anticorps, IgG ou F(ab')2, aux protéines totales, à l'activité anticorps et au rendement en éléments à activité anticorps.

Les tableaux 1 et 2 donnent l'ensemble des résultats obtenus à partir respectivement du premier et du second exemples chiffrés d'extraction de la caséine, visés ci-dessus.

Les fractions sérocolostrales hypoallergéniques et à forte activité anticorps de l'invention sont utilisables comme complément nutritionnel dans le domaine de la diététique. Elles sont également utilisables pour la préparation de médicaments destinés à la prophylaxie et aux traitements par voie orale des diarrhées animales ou humaines , de toutes origines bactérienne, virale ou parasitaire.

L'avantage que présente la fraction sérocolostrale de l'invention dans cette utilisation réside dans son caractère hypoallergénique. Il importait en effet que l'administration chez l'homme d'un médicament de ce type n'entraîne pas de réactions allergéniques dues à la β-lactoglobuline, et également à la sérumalbumine, à l'α-lactalbumine et enfin aux fragments Fc. Les anticorps bovins étant structurellement différents des anticorps humains principalement au niveau des fragments Fc, ceux-ci peuvent se comporter comme un antigène supplémentaire. Cette considération est importante si l'on prend en compte le fait qu'au cours des entérites , l'épithélium intestinal est fragilisé et laisse mieux passer les allergènes d'origine alimentaire. Dans la fraction sérocolostrale de l'invention, le fragment F(ab')2 qui est obtenu par hydrolyse ménagée des IgG par la pepsine, conserve intact ses sites de combinaison antigénique, tout en ayant une immunogénicité réduite.

La présente invention n'est pas limitée aux modes de réalisation qui viennent d'être décrits à titre d'exemples non exhaustifs. En particulier le colostrum de départ peut provenir des vaches hyper-immunisées , ce qui confère à la fraction sérocolostrale obtenue un caractère anticorps spécifique complémentaire.

**TABLEAU 1**

| | Quantité (Kg) | MS (Kg) | IgG ou F(ab')2 (Kg) | Pr (Kg) | Activité (10⁻⁴ mol/100gPr) IgG \| F(ab')2 | Rendement en IgG ou F(ab')2 (%) |
|---|---|---|---|---|---|---|
| Colostrum écrémé | 39,63 | 9,03 | 2,66 | 4,95 | 3,6 | |
| | | Dilution et précipitation acide des caséines | | | | 65 |
| Sérocolostrum dilué 5 | 249,9 | 4,52 | 1,74 | 2,39 | 4,9 | |
| | | Concentration par ultrafiltration | | | | 100 |
| Sérocolostrum concentré | 59,9 | 3,29 | 1,82 | 2,35 | 5,2 | |
| | | Hydrolyse ménagée par la pepsine | | | | 100 |
| Sérocolostrum hydrolysé | 61,3 | 3,66 | 1,21 | 2,66 | 4,6 | |
| | | Adsorption de la β-lactoglobuline | | | | 29 |
| Sérbolostrum hydrolysé hypoallergénique | 67,2 | 1,48 | 0,33 | 0,89 | 3,7 | |
| | | Séchage par atomisation | | | | 97 |
| Poudre | 1,23 | 1,14 | 0,32 | 0,76 | 4,2 | |

**TABLEAU 2**

| | Quantité (Kg) | MS (Kg) | IgG ou F(ab')2 (Kg) | Pr (Kg) | Activité (10⁻⁴ mol/100g Pr) IgG \| F(ab')2 | Rendement en IgG ou F(ab')2 (%) |
|---|---|---|---|---|---|---|
| Colostrum écrémé | 78,17 | 17,43 | 3,17 | 10,85 | 1,95 | |
| | | Extraction des caséines par la présure | | | | 96 |
| Sérocolostrum | 65,55 | 9,91 | 2,9 | 4,94 | 3,95 | |
| | | Hydrolyse ménagée par la pepsine | | | | 89 |
| Sérocolostrum hydrolysé | 70,3 | 10,68 | 1,73 | 5,19 | 3,3 | |
| | | Dilution puis adsorption de la β-lactoglobuline | | | | 35 |
| Sérocolostrum hydrolysé hypoallergénique | 181,5 | 6,85 | 0,60 | 2,08 | 2,9 | |
| | | Séchage par atomisation | | | | 87 |
| Poudre | 4,58 | 4,33 | 0,52 | 1,79 | 2,9 | |

## Revendications

1. Fraction sérocolostrale hypoallergénique d'origine bovine à forte activité anticorps **caractérisée en ce qu'**elle est exempte de β-lactoglobuline, de α-lactalbumine, de sérum-albumine et de fragments Fc dès IgG, **en ce que** les éléments à activité anticorps sont les fragments F(ab')2 des IgG et **en ce que** le nombre de moles de F(ab')2 est d'au moins 2,5.10⁻⁴ pour 100 g de protéines totales.

2. Fraction sérocolostrale selon la revendication 1 **caractérisée en ce qu'**elle se présente sous forme de liquide, liquide congelé ou poudre.

3. Fraction sérocolostrale selon l'une des revendications 1 et 2 obtenue à partir de colostrum hyperimmun.

4. Utilisation de la fraction sérocolostrale selon la revendication 1 comme complément nutritionnel, notamment dans le domaine de la diététique.

5. Utilisation de la fraction sérocolostrale selon la revendication 1 pour l'obtention d'un médicament destiné à la prophylaxie ou au traitement, par voie orale, des diarrhées animales ou humaines.

6. Procédé pour la préparation de la fraction sérocolostrale de la revendication 1 **caractérisé en ce qu'**il consiste à extraire les caséines d'un colostrum bovin écrémé, à faire une hydrolyse contrôlée du sérocolostrum ainsi obtenu, par la pepsine, et à adsorber la β-lactoglobuline contenue dans l'hydrolysat par mise en contact de celui-ci , sous agitation pendant une durée déterminée, avec une résine échangeuse d'ions anionique faible de type amine tertiaire.

7. Procédé selon la revendication 6 **caractérisé en ce que** l'adsorption de la β-lactoglobuline par mise en contact avec la résine anionique est réalisée en maintenant le pH à une valeur de l'ordre de 6 , notamment par addition d'acide chlorhydrique.

8. Procédé selon la revendication 7 **caractérisé en ce que** la quantité. de résine anionique qui est utilisée est de l'ordre de 1 Kg de résine humide pour 2 litres d'hydrolysat de sérocolostrum à environ 50 g par litre de matière sèche.

9. Procédé selon la revendication 6 **caractérisé en ce que** l'hydrolyse ménagée du sérocolostrum se déroule dans les conditions opératoires suivantes :
- pH régulé entre 3,5 et 4,5
- température comprise entre 3 et 40°C
- durée comprise entre 3 et 8 heures
- quantité de pepsine , mesurée en unités SI par milligramme de protéine, comprise entre 1033 et 3400 (entre 31 et 102 unités Anson).

10. Procédé selon la revendication 9 **caractérisé en ce que** les conditions opératoires retenues sont les suivantes : pH 3,8 ; 37°C pendant 4 heures avec une quantité de pepsine correspondant à 1700 unités SI (51 unités Anson) par milligramme de protéine.

## Patentansprüche

1. Hypoallergene Rinder-Serocolostral-Fraktion mit hoher Antikörper-Aktivität, **dadurch gekennzeichnet,**
**dass** sie frei von β-Lactoglobulin, α-Lactalbumin, Serum-Albumin und Fc-Fragmenten der IgG ist,
**dass** die Elemente mit Antikörper-Aktivität die Fragmente F(ab')₂ der IgG sind und
**dass** die Anzahl der Mole von F(ab')₂ mindestens 2,5.10⁻⁴ beträgt auf 100 g Gesamtproteine.

2. Serocolostral-Fraktion nach Anspruch 1, **dadurch gekennzeichnet, dass** sie in Form einer Flüssigkeit, einer gelierten Flüssigkeit oder eines Pulvers vorliegt.

3. Serocolostral-Fraktion nach einem der Ansprüche 1 und 2, die aus hyperimmunem Colostrum hergestellt ist.

4. Verwendung der Serocolostral-Fraktion nach Anspruch 1 als Nahrungsergänzungsmittel, insbesondere auf dem Gebiet der Diätetik.

5. Verwendung der Serocolostral-Fraktion nach Anspruch 1 zur Herstellung eines Arzneimittels für die Prophylaxe oder Behandlung auf oralem Wege von Tier- oder Human-Diarrhöen.

6. Verfahren zur Herstellung der Serocolostral-Fraktion nach Anspruch 1, **dadurch gekennzeichnet, dass** man aus einem entrahmten Rinder-Colostrum die Caseine extrahiert, mit dem dabei erhaltenen Serocolostrum eine kontrollierte Hydrolyse mit Pepsin durchführt und das in dem Hydrolysat enthaltene β-Lactoglobulin an einem schwachen Anionenaustauscherharz vom tertiären Amin-Typ adsorbiert, indem man es unter Rühren für eine vorgegebene Zeitspanne damit in Kontakt bringt.

7. Verfahren nach Anspruch 6, **dadurch gekennzeichnet, dass** man die Adsorption des β-Lactoglobulins durchführt, indem man es mit dem anionischen Harz in Kontakt bringt unter Aufrechterhaltung des pH-Wertes bei einem Wert in der Größenordnung von 6, insbesondere durch Zugabe von Chlorwasserstoffsäure.

8. Verfahren nach Anspruch 7, **dadurch gekennzeichnet, dass** die verwendete Menge des anionischen Harzes in der Größenordnung von 1 kg feuchtem Harz auf 2 l Serocolostrum-Hydrolysat mit etwa 50 g/l Trockensubstanz liegt.

9. Verfahren nach Anspruch 6, **dadurch gekennzeichnet, dass** die kontrollierte Hydrolyse des Serocolostrums unter den folgenden Bedingungen durchgeführt wird:
- bei einem auf einem Wert zwischen 3,5 und 4,5 eingestellten pH-Wert,
- bei einer Temperatur zwischen 3 und 40 °C,
- für eine Dauer zwischen 3 und 8 h,
- mit einer Pepsinmenge, gemessen in Si-Einheiten pro mg Protein, zwischen 1033 und 3400 (zwischen 31 und 102 Anson-Einheiten).

10. Verfahren nach Anspruch 9, **dadurch gekennzeichnet, dass** die aufrechterhaltenen Verfahrensbedingungen die folgenden sind:
pH-Wert von 3,8; 37 °C für eine Dauer von 4 h mit einer Pepsin-Menge entsprechend 1700 SI-Einheiten (51 Anson-Einheiten) pro Milligramm Protein.

## Claims

1. Hypoallergenic serocolostral fraction of bovine origin with high antibody activity, **characterised in that** it is free from β-lactoglobulin, α-lactalbumin, serum-albumin and Fc fragments of IgG, **in that** the antibody activity elements are F(ab')2 fragments of IgG, and **in that** the number of mols of F(ab')2 is at least 2.5x10(-4) to 100 g of proteins in total.

2. Serocolostral fraction according to Claim 1, **characterised in that** it is in liquid, frozen liquid or powder form.

3. Serocolostral fraction according to one of the Claims 1 and 2, obtained from hyperimmune colostrum.

4. Using the serocolostral fraction according to Claim 2 as a nutritional complement, particularly in the field of dietetics.

5. Using the serocolostral fraction according to Claim 1 to obtain a medicament intended for prophylaxis or treatment by mouth of animal or human diarrhoea.

6. Process for preparing the serocolostral fraction in Claim 1, **characterised in that** it consists in extracting the caseins of a skimmed bovine colostrum, in carrying out controlled hydrolysis of the serocolostrum thus obtained, by pepsin, and in adsorbing the β-lactoglobulin contained in the hydrolysate by putting it in contact, by stirring for a predetermined time, with a low anionic ion-exchange resin of the tertiary amine type.

7. Process according to Claim 6, **characterised in that** the β-lactoglobulin is adsorbed by coming into contact with the anionic resin while keeping the pH at a figure in the region of 6, particularly by adding hydrochloric acid.

8. Process according to Claim 7, **characterised in that** the quantity of anionic resin which is used is in the region of 1 kg of moist resin to 2 litres of serocolostrum hydrolysate at around 50 g per litre of dry material.

9. Process according to Claim 6, **characterised in that** the hydrolysis made of the serocolostrum develops under the following operating conditions:
- pH regulated between 3.5 and 4.5
- temperature between 3 and 40°C
- time between 3 and 8 hours
- quantity of pepsin, measured in SI units per milligram of protein, between 1033 and 3400 (between 31 and 102 Anson units).

10. Process according to Claim 9, **characterised in that** the operating conditions decided on are as follows: pH 3.8; 37°C for 4 hours with a quantity of pepsin corresponding to 1700 SI units (51 Anson units) per milligram of protein.
